(19) Europäisches Patentamt
European Patent Office
Office européen des brevets

(11) Numéro de publication: **0 393 106 B1**

(12) **FASCICULE DE BREVET EUROPEEN**

(45) Date de publication de fascicule du brevet: **06.04.94**

(51) Int. Cl.5: **C12P 19/00**, A61K 31/725, A23L 1/308, A61K 35/78

(21) Numéro de dépôt: **89900265.3**

(22) Date de dépôt: **02.12.88**

(86) Numéro de dépôt internationale : **PCT/FR88/00592**

(87) Numéro de publication internationale : **WO 89/05354 (15.06.89 89/13)**

(54) **NOUVEAUX COMPLEXES MACROMOLECULAIRES D'ORIGINE VEGETALE LEUR PROCEDE DE PREPARATION ET LEUR UTILISATION A TITRE D'AGENT HYPOCHOLESTEROLEMIANT.**

(30) Priorité: **02.12.87 FR 8716734**

(43) Date de publication de la demande: **24.10.90 Bulletin 90/43**

(45) Mention de la délivrance du brevet: **06.04.94 Bulletin 94/14**

(84) Etats contractants désignés: **AT BE CH DE FR GB IT LI LU NL SE**

(56) Documents cités:
**US-A- 2 754 214**
**US-A- 4 483 875**

**Journal of Applied Biochemistry, vol. 2,1980, Academc Press, Inc., F.G.J. Voragen et al.: "Solubilization of apple cell walls with poly-saccharide-degrading enzymes"**

(73) Titulaire: **ASSOCIATION POUR LE DEVELOP-PEMENT DE LA RECHERCHE EN VUE DE PROMOUVOIR LES PRODUCTIONS AGRICO-LES**
**DANS LE DEPARTEMENT DE LA MANCHE (ADERA-MANCHE)**
**Hotel du Département de la Manche**
**F-50008 Saint-Lo(FR)**

(72) Inventeur: **BARON, Alain**
**4, rue de Rennes**
**F-35132 Vezin-le-Coquet(FR)**

(74) Mandataire: **Phélip, Bruno et al**
**c/o Cabinet Harlé & Phélip**
**21, rue de La Rochefoucauld**
**F-75009 Paris (FR)**

## Description

La présente invention concerne de nouveaux complexes macromoléculaire d'origine végétale, qui consistent notamment en fractions pariétales de pommes ou de sous-produits de la pomme, dont la structure moléculaire n'a pas été altérée. Elle concerne également un procédé d'obtention de ces nouveaux complexes par hydrolyse enzymatique d'une matière première à base de pommes ou d'un sous-produit des divers traitements effectués sur les pommes. Enfin, elle concerne l'application de ces complexes macromoléculaires à titre d'agents hypocholestérolémiant, dans les domaines alimentaire, diététique et pharmaceutique.

De façon plus particulière, la présente invention s'inscrit dans le cadre de la revalorisation des résidus et sous-produits industriels de la transformation des pommes. De tels résidus sont très nombreux dans l'industrie cidricole.

Le traitement des pommes en vue de l'élaboration de boissons (jus, cidre, eau de vie de cidre), de concentrés ou de compotes, s'accompagne de la production d'un certain nombre de résidus dont l'élimination ou la revalorisation n'est pas toujours aisée. Le plus abondant de ceux-ci est le marc, obtenu après extraction du moût par pressurage de la pulpe ayant, le cas échéant, subi un traitement enzymatique préalable à l'aide de "pectinases" ; le plus souvent, le marc est ensuite totalement désucré par diffusion d'eau à contre-courant. Il est alors destiné, soit à l'épandage, soit à l'alimentation animale, soit, pour les meilleurs, à la pectinerie. D'autres déchets proviennent de la clarification du moût sorti de presse. Il s'agit, soit des rétentats d'ultrafiltration ou de microfiltration tangentielle, soit des gels de pectate de calcium ("chapeaux bruns" de défécation ou de flottation), soit des "boues" de collage à la gélatine ou à la bentonite. Ces produits, après décantation ou essorage, sont envoyés à l'épandage. Enfin, un dernier type de sous-produit de l'industrie est constitué par les résidus de pectinerie, résultats de l'extraction des pectines du marc. Tous ces résidus sont caractérisés par une teneur importante en macromolécules d'origine pariétale. La présente invention offre le moyen d'extraire, de l'un ou l'autre de ces produits, les fractions macromoléculaires qui ont un effet bénéfique sur le métabolisme du cholestérol.

En effet, de nombreuses publications ont montré que le régime alimentaire permet de contrôler la chlolestérolémie (PORTMAN et STARE, 1959, Physiol. rev. 39, 407-410). Le rôle des pectines dans la diminution du taux de cholestérol sérique et hépatique chez l'animal et chez l'homme est largement rapporté par de nombreux auteurs. Toutefois, les conditions expérimentales et les résultats obtenus sont très variables, tant chez l'homme que chez l'animal, essentiellement le rat. (On se reportera, par exemple, à la présentation bibliographique de BEHALL et REISER, 1986, in Chemistry and Function of Pectins, FISHMAN et JEN, éd., A.C.S. Pub., 248-265). Ces expérimentations mettent en oeuvre, le plus souvent, des pectines non caractérisées. Seuls, quelques auteurs ont essayé de caractériser la pectine qu'ils utilisent. Depuis 1978-1979, en effet, plusieurs travaux ont tenté de mettre en évidence une corrélation entre l'action de la pectine et sa structure globale (degré de méthoxylation et masse moléculaire). Dans tous les cas, les effets observés, lorsqu'ils existent, sont obtenus à des doses de pectine correspondant à un équivalent de pommes de 1 à 3 kg/jour. Or, des rations de 2 à 3 pommes par jour chez l'homme (environ 2 g de pectine) suffisent pour agir sur l'hypercholestérolémie (CANELLA et al., 1962, Arcispedale S. Anna de Ferrara, 15, 803-814 ; SABLE-AMPLIS et al., 1980, 22nd Int. Conf. on Biochem. of Lipids. Milan; SABLE-AMPLIS et al., 1983. Nutr. Res., 3, 325-328). Des résultats expérimentaux spectaculaires ont été obtenus chez l'animal. Chez des hamsters qui augmentent exagérément le taux de cholestérol circulant avec l'âge, l'ingestion régulière de pommes en même temps que l'aliment standard, empêche cette évolution anormale et corrige tous les troubles métaboliques qui lui sont associés (SABLE-AMPLIS et al., 1979, Nutr. Rep. Int., 19, 723-732). Le même régime réduit fortement l'hypercholestérolémie induite par un régime athérogène chez d'autres espèces (cobayes, lapins). L'apport de pectines commerciales extraite de végétaux dans la ration de base du hamster (7% du régime) n'a comparativement que peu d'effets (TSAI A.C., ELIAS J., KELLEY J.J., LIN R.S.C. et ROBSON J.R.K., 1976, J. Nutr. Vol. 106, pages 118-123).

Ainsi, l'effet hypocholestérolémiant de la pomme paraît dû à des composés associés à la paroi végétale ; cependant, la pectine, telle qu'elle en est extraite, (par exemple par hydrolyse acide et précipitation en milieu alcoolique) n'est pas la fraction la plus active. Ceci peut rendre compte de l'observation faite par GINTER et col. (1983, Nutr. Rep. Int., 27, 1049-1057) : des pectines parfaitement purifiées ayant des degrés d'estérification compris entre 0 et 91% n'ont aucun pouvoir hypocholestérolémiant chez le cochon d'Inde, alors que la pectine initiale brute (6% du régime) est significativement active.

La présente invention permet, au contraire, d'obtenir un complexe macromoléculaire insoluble dans les milieux aqueux et dans lequel la pectine (déterminée par dosage de l'acide galacturonique) n'est pas prépondérante, un tel complexe présentant l'activité hypocholestérolémiante recherchée. Les expériences menées par les inventeurs ont montré que les effets de la fraction active selon l'invention étaient supérieurs

EP 0 393 106 B1

à ceux obtenus avec des taux de pectines allant bien au-delà de celui représenté par ladite fraction dans le régime alimentaire.

On soulignera à ce propos que le but poursuivi ici est de dégrader la paroi des cellules végétales pour n'en recueillir que les fractions présentant l'activité recherchée, ce qui constitue une différence essentielle avec les fractions décrites dans le brevet européen n° 35643, suivant lequel on a cherche à obtenir des cellules dont la paroi doit rester intacte.

Les complexes macromoléculaires d'origine végétale selon la présente invention (ci-après désignés également par l'expression "fraction active selon l'invention") sont caractérisés par le fait qu'ils renferment, sur une base sèche,

- de 5 à 25% en poids de polyosides cationiques ;
- de 5 à 15% en poids d'hémicelluloses ;
- de 0 a 50% en poids de cellulose ;
- de 25 à 55% en poids de pigments quinoniques polymérisés.

Les polyosides cationiques sont notamment des dérivés des pectines. Celles-ci sont en faible quantités dans la fraction active selon l'invention, et elles ont subi l'action de la pectinestérase ; on diminue ainsi la quantité de méthanol saponifiable (par la flore colique notamment, lors de l'ingestion des végétaux ou de leurs extraits riches en pectines natives). A cet égard, la fraction active peut être ingérée, en quantité plus importante que son équivalent pomme, sans augmenter les risques liés au méthanol.

Apparaît ici une différence essentielle entre la fraction active de l'invention et les produits décrits dans le brevet européen n° 89 056, pour lesquels on a cherché à conserver les pectines natives hautement méthylées et en quantité la plus élevée possible.

Les monomères prépondérants des hémicelluloses sont l'arabinose, le galactose, le glucose et le mannose.

La proportion relative des constituants principaux des fractions selon l'invention peut varier selon l'origine de la matière première et les conditions de l'hydrolyse enzymatique, comme cela sera décrit ci-après.

Lors de l'ingestion de ces complexes, des effets métaboliques importants sont observés.

- Au niveau sérique, on observe une augmentation du taux des HDL (Lipoprotéines à densité élevée), s'accompagnant le plus souvent d'une diminution de la cholestérolémie chez les sujets hypercholestérolémiques.
- Au niveau hépatique, chez l'animal, on note une diminution du stockage du cholestérol et une réduction de la concentration des esters de cholestérol, liées à une baisse de la vitesse d'estérification du cholestérol néosynthétisé. Ceci s'accompagne d'une production accrue d'acides biliaires favorisée par une activité cholestérol 7a-hydroxylase stimulée.
- Au niveau tissulaire périphérique, les teneurs en cholestérol baissent, et le rapport des cholestérol estérifié/non estérifié décroît, indiquant une diminution des formes de stockage du cholestérol dans les tissus. Ceci est particulièrement intéressant à retenir étant donné le rôle important que joue l'estérification du cholestérol dans la génèse de l'athérosclérose.

La présente invention a également pour objet un procédé de préparation des complexes macromoléculaires d'origine végétale, tels que définis ci-dessus, caractérisé par le fait qu'il consiste :

- à effectuer une hydrolyse enzymatique d'une matière première à base de pomme, à l'état hydraté, a l'aide d'une préparation enzymatique contenant des activités pectinolytiques, hémicellulosiques et/ou cellulosiques ;
- à séparer le produit de la réaction du milieu réactionnel ;
- à en recueillir une fraction entre des tamis de 0,5 à 0,05 mm d'ouverture de maille ; puis,
- à purifier éventuellement et à sécher cette fraction.

En particulier, on utilise, comme matière première à base de pomme, des résidus à teneur importante en macromolécules d'origine pariétale :

- le marc, humide ou sec (que l'on réhydrate alors dans ce cas) ;
- les rétentats d'ultrafiltration et de microfiltration, obtenus dans le traitement industriel des pommes ;
- les gels de pectate de calcium (rétentats d'ultrafiltration et chapeaux bruns de défécation); et
- les résidus de pectinerie.

Pour la mise en oeuvre de l'hydrolyse enzymatique, on utilise avantageusement une préparation enzymatique qui contient des activités pectinestérase, polygalacturonase, cellulase (CMC) et arabinogalactanase.

Les quantités d'enzymes à ajouter varient selon le type de la matière première. Ainsi, l'utilisation des rétentats d'ultrafiltration ou des chapeaux de flottation ne nécessite pas la présence de cellulases. Au contraire, dans le cas du marc, on préconise l'emploi de cellulases en proportion importante.

3

Par ailleurs, la vitesse de réaction est proportionnelle à la quantité d'enzymes utilisée. Ainsi, les doses d'enzymes à ajouter sont à déterminer selon des critères économiques prenant en compte les deux facteurs = temps du traitement, coût de l'enzyme.

Les exemples ci-après donnent les quantités utilisables.

Dans l'industrie des boissons à base de fruits, les enzymes, désignées sous le vocable "pectinases", sont employées, soit pour le traitement de la pulpe, de façon à faciliter l'expression du jus (c'est la règle pour les petits fruits rouges ou pour les pommes surmaturées), soit pour la clarification des moûts par l'une ou l'autre des techniques déjà citées. Ces enzymes sont également de plus en plus utilisées pour l'élaboration des nectars (pectinases macérantes) ou pour l'obtention de jus végétaux, en particulier des fruits tropicaux, sans intervention de presse (pectinases liquéfiantes). Pour chacune des applications de ces enzymes, il est évident que le spectre des activités nécessaires est différent : par exemple, pour la clarification par flottation, l'enzyme doit être riche en activité pectinestérase (PE) et ne pas contenir d'activité polygalacturonase (PG) ou pectinelyase (PL) ; au contraire, pour obtenir la macération d'un tissu, il convient d'avoir une PG sans activité PE ; le traitement des pulpes avant pressurage, nécessite des activités PE, PG, arabanases et galactanase dans des rapports appropriés, etc. Ainsi, l'homme du métier se voit proposer, par les fabricants, des préparations de "pectinases" fort différentes selon le substrat et la technologie mise en oeuvre.

Dans le cas de la présente invention, il sera à la portée de l'homme du métier de choisir, parmi les préparations enzymatiques proposées dans le commerce, celles qui conviennent pour les utiliser notamment en mélange, afin d'orienter et de contrôler l'hydrolyse enzymatique en vue de l'obtention du complexe pariétal actif. Les exemples donnés ci-dessous apportent des précisions quant aux enzymes utilisées selon l'origine de la matière première. D'une façon générale, les conditions de la réaction correspondent à celles de l'action optimale des enzymes, à savoir pH compris entre 4 et 5 et une température inférieure à 50°C, notamment comprise entre 35 et 50°C.

Après l'hydrolyse enzymatique, on recueille la fraction active par exemple par tamisages successifs de l'hydrolysat.

On peut également recueillir la fraction active par ultrafiltration.

Ensuite, on purifie cette fraction active par exemple par diafiltration, avec éventuellement addition d'agents chélateurs d'ions (par exemple, l'oxalate d'ammonium), pour appauvrir l'extrait en matière minérale.

Après séchage, par un procédé ou un autre, on obtient des produits se présentant sous forme d'une poudre colorée en brun. Ces produits sont thermostables et peuvent être dispersés dans des phases aqueuses. On peut également les placer sous forme de poudres, de tablettes ou de granulés.

La présente invention porte également sur l'utilisation des complexes macromoléculaires tels que définis ci-dessus, à titre d'agent hypocholestérolémiant. En particulier, l'invention porte sur des compositions alimentaires ou diététiques ou des boissons renfermant une quantité des complexes macromoléculaires définis ci-dessus, qui est suffisante pour produire un effet hypocholestérolémiant. De même, l'invention porte sur des compositions pharmaceutiques hypocholestérolémiantes, renfermant une quantité efficace des complexes macromoléculaires tels que définis ci-dessus.

L' incorporation des complexes selon l'invention dans les produits alimentaires et diététiques ne présente aucune difficulté. Ainsi, on peut les incorporer dans des farines, des produits laitiers, des boissons troubles ou pulpeuses, ainsi que dans les spécialités diététiques, dans les différents domaines de l'industrie alimentaire : céréales, biscuiterie, boulangerie, produits laitiers, boissons, confiserie, etc.

Comme indiqué ci-dessus, les fractions de l'invention peuvent être ingérées directement sous les formes indiquées de poudres, tablettes et granulés.

Pour illustrer davantage l'objet de la présente invention, on va décrire maintenant, à titre d'exemples purement illustratifs et non limitatifs, plusieurs modes de réalisation.

Dans cette partie relatant les expériences effectuées par les inventeurs, les quantités d'enzymes sont données en unité enzymatique ; celle-ci est définie comme la quantité d'enzyme qui transforme une $\mu$mole ou un microéquivalent de substrat par minute. L'activité pectinestérase (PE) sur une solution de pectine (DE 75) à 5g/l est déterminée a 30°C et pH 4,5, a l'aide d'un pHstat. L'activité des dépolymérases est mesurée par dosage des groupements réducteurs formés par hydrolyse du substrat polysaccharidique. On utilise la technique en continu au cyanoacétamide décrite par BARON et al. dans un article accepté pour publication dans LWT de 1987 ou 1988). Les méthodes analytiques concernant la composition des fractions sont celles communément employées au laboratoire et décrites dans la bibliographie.

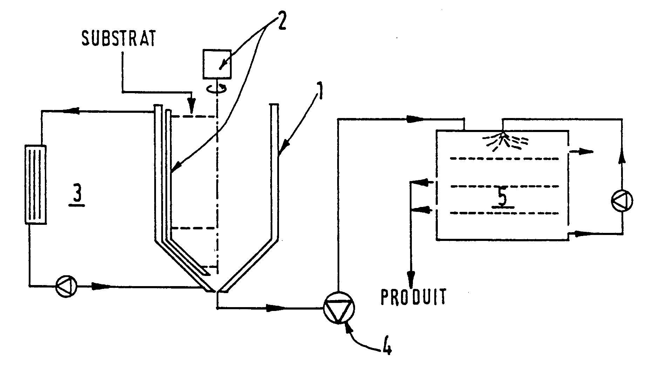

Pour la mise en oeuvre des exemples 2 et 3, on a utilisé l'appareillage représenté de façon schématique sur la figure unique du dessin annexé.

4

Cet appareillage comprend une cuve d'enzymage 1 à double enveloppe à circulation d'eau, qui est équipée d'un système d'homogénéisation 2 à racleur et vis creuse, ainsi que d'un système de régulation thermique 3 ; la vitesse de rotation, ajustable, est fixée à 30 rpm. Le produit en cours d'hydrolyse est prélevé à la base de la cuve 1 à l'aide d'une pompe 4 (PCM) et envoyé sur une tamiseuse vibrante 5. On recueille dans les tamis intermédiaires (0,180 et 0,053 mm) la fraction active.

Exemple 1

Dans cet exemple, la matière première utilisée est de la râpure de pomme provenant d'un mélange de cultivars utilisé traditionnellement en cidrerie.

Obtention de la fraction active :

La râpure (1 tonne) est enzymée à la température ambiante, à l'aide d'un mélange de "pectinases", de façon à avoir les activités suivante :

| | |
|---|---|
| - Pectinestérase | 37 U/kg (sur une base sèche) |
| - Polygalacturonase | 15 U/kg |
| - Cellulase (CMC) | 11 U/kg |

On laisse agir l'enzyme pendant une heure, sans agitation. On procède alors au pressurage de la râpure. Le jus (820 1) est tamisé (à travers un tamis de 0,5 mm d'ouverture de maille), pour retenir les fragments de pulpes. Il est préchauffé et envoyé sur un module d'ultrafiltration (modèle Abcor, avec une membrane d'ultrafiltration dont le seuil de coupure est de 20 000 daltons) ; la température est maintenue entre 45 et 50 °C, de façon à diminuer la viscosité sans dénaturer les enzymes qui sont passées dans le jus. (Le jus clarifié est retourné dans le circuit normal de la cidrerie). Les fractions macromoléculaires actives, retenues par la membrane, sont ainsi progressivement concentrées environ dix fois. On procède alors à une dialyse par diafiltration pour éliminer de façon exhaustive les petites molécules (sucres, acides aminés, acides organiques, sels libres ...) Le rétentat est concentré à nouveau deux fois. Le produit actif peut être recueilli, soit simplement par séchage (lyophilisation par exemple), soit, selon une variante, précipité par un alcool (éthanol ou isopropanol à 80 °GL final), décantation du précipitat, redispersion dans un minimum d'eau et séchage comme précédemment. Cette deuxième variante limite les quantités d'eau à évaporer. Dans les conditions de cet exemple, 760 g d'extraits sont ainsi obtenus.

Résultats :

L'analyse des principaux constituants de cette fraction donne les résultats suivants :

| | |
|---|---|
| - Pectines | 247,8 g/kg sec |
| - Hémicelluloses | 58,4 g/kg sec |
| - Cellulose et pigments | 639,0 g/kg sec |

Application :

Ce produit est incorporé à raison de 0,65% (en poids sec) dans un aliment pour hamster. La teneur en pectine de l'aliment n'est que de 0,16%. Parmi les effets de cet extrait, les plus remarquables sont une diminution du cholestérol total au niveau du foie (de 22%) et des tissus périphériques (de 12%, dans l'aorte), et, surtout, une diminution importantes des formes estérifiées (de 28%, dans le foie par exemple), entraînant une baisse du rapport de cholestérol estérifié/cholestérol libre (de 48,3% dans la paroi du caecum).

Exemple 2

Cet exemple décrit une forme simplifiée de l'invention, conduisant aisément à l'obtention d'un produit enrichi en fractions actives, mais contenant encore une proportion importante de substances inactives.

Obtention :

Le marc sec (10 kg), tel qu'il est produit en cidrerie, est réhydraté par cinq à six fois son poids en eau, éventuellement préchauffé (50-60°C) et alcalinisé par une petite quantité de solution basique (NaOH, KOH, $Na_2CO_3$,etc.) On ajuste le pH du milieu entre 4 et 5, et on maintient la température à 45°C ± 5 (par exemple, à l'aide d'une cuve à double enveloppe à circulation d'eau). On procède alors à l'enzymage du marc de façon à apporter les activités suivantes :

```
- Pectinestérase .......... 14'0 U/kg de marc sec
- Polygalacturonase ....... 500 U/kg  "  "     "
- Cellulase ............... 450 U/kg  "  "     "
- Arabinogalactanase ...... 30 U/kg   "  "     "
```

Après 12 heures d'action, on élimine les produits d'hydrolyse par une succession de pressurages et de retrempages des gâteaux de presse par de l'eau. Cette opération de remiage peut être remplacée par une diffusion d'eau à contre-courant. Après un quatrième pressurage, le résidu contenant les fractions pariétales actives est séché dans une étuve ventilée (55°C) ou par tout autre moyen. Il est finalement broyé a l'aide d'un broyeur à galets.

Résultats :

Sous cette forme, le produit contient une part importante de ballast inactif (provenant, en particulier, des tissus épidermiques, des faisceaux ligneux, des parois des loges carpellaires, des pépins). Pour 1 kg de marc sec mis en jeu, on a recueilli ainsi 0,60 kg de produit. L'analyse de sa composition donne les résultats suivants :

| | |
|---|---|
| - Pectines | 74,3 g/kg sec |
| - Hémicelluloses | 96,3 g/kg sec |
| - Cellulose | 375 g/kg sec |
| - Pigments | 305 g/kg sec |
| - Azote organique | 91,2 g/kg sec |
| - Matière grasse | 49,0 g/kg sec |
| - Matière minérale | 7,6 g/kg sec |

Application :

Pour contrôler l'efficacité métabolique de ce produit, la poudre obtenue est incorporée, à raison de 12% en poids, dans un régime pour hamster, sur la base de la teneur en cellulose (5% final).

L'action de cette fraction est testée sur deux populations de hamsters, l'une à cholestérolémie normale, l'autre rendue hypercholestérolémique par ajout de 0,1% de cholestérol dans le régime. Les effets de la fraction sur la cholestérolémie sont donnés dans le tableau suivant.

| | Régime sans la fraction | | Régime avec la fraction acive | |
|---|---|---|---|---|
| | Animal sain | Animal hypercholestérolémique | Animal sain | Animal hypercholestérolémique |
| Chol. total (mmoles/1) | 4,06 | 6:72 | 3,66 | 4,35 |
| Chol. libre (mmoles/1) | 1,32 | 2,00 | 1,13 | 1,33 |
| Triglycérides (mmoles/1) | 2,57 | 2,63 | 2,41 | 1,79 |

Il apparaît que la fraction active réduit la cholestérolémie des animaux sains (de 9,8%) et surtout celle des animaux hypercholestérolémiques (de 35%) ; ceux-ci retrouvent pratiquement une cholestérolémie normale (+7%). On observe également une action de la fraction sur les triglycérides sériques.

Dans ces essais, la pectine ne représente que 1% du régime alimentaire. Cependant, les effets de la fraction sont supérieurs à ceux obtenus avec des taux de pectines aussi élevés que 10%. Ainsi, la fraction active obtenue associe à la pectine les autres constituants pariétaux (polyosides neutres et pigments quinoniques).

Exemple 3

Cet exemple illustre un procédé de l'invention qui permet d'obtenir la fraction active débarrassée du ballast.

Obtention :

Comme dans l'exemple précédent, on utilise du marc de pomme enzymé par le mélange suivant :

```
        - Pectinestérase ........... 4100 U/kg de marc sec
        - Polygalacturonase ........ 9300 U/kg  "    "    "



        - Cellulase (CMC) .......... 6900 U/kg  "    "    "
        - Arabinogalactanase ....... 200 U/kg  "    "    "
```

A la fin de l'hydrolyse ayant duré pendant 15 heures, le milieu réactionnel est dilué de moitié par addition d'eau. La pulpe est reprise par la pompe, et elle est envoyée sur une tamiseuse vibrante équipée d'une série de tamis superposés dont les dimensions d'ouverture de maille sont successivement de 0,5 mm, 0,125 mm et 0,053 mm. En fin de tamisage, on procède à une aspersion des tamis par de l'eau contenant éventuellement un chélateur d'ions. La fraction active est recueillie dans les tamis de mailles 0,125 et 0,053 mm. Après séchage, le produit attendu se présente comme une poudre fine de couleur brune. Pour 1 kg de marc mis en oeuvre, on recueille 204 g de produit.

Résultats :

L'analyse de la fraction ainsi obtenue donne les résultats suivants :

| | |
|---|---|
| - Pectine | 49,3 g/kg sec |
| - Hémicellulose | 94,6 g/kg sec |
| - Cellulose et pigments | 730 g/kg sec |
| - Azote organique | 37 g/kg sec |
| - Matière grasse | - |
| - Matière minérale | 5,2 g/kg sec |

La succession des tamis utilisés est celle qui, à l'expérience, a permis d'obtenir le meilleur débit de tamisage.

Application :

Parmi les effets métaboliques observés, signalons, au niveau sérique, une augmentation de 15% des HDL.

**Revendications**

1. Complexes macromoléculaires d'origine végétale, caractérisés par le fait qu'ils renferment, sur une base sèche,
   - de 5 à 25% en poids de palyosides cationiques ;

7

- de 5 à 15% en poids d'hémicelluloses;
- de 0 à 50% en poids de cellulose ;
- de 25 à 55% en poids de pigments quinoniques polymérisés.

2. Complexes macromoléculaires selon la revendication 1, caractérisés par le fait que les polyosides cationiques sont essentiellement constitués par des dérivés de pectines.

3. Complexes macromoléculaires selon l'une des revendications 1 et 2, caractérisés par le fait que les monomères prépondérants des hémicelluloses sont l'arabinose, le galactose, le glucose et le mannose.

4. Procédé de préparation des complexes macromoléculaires d'origine végétale, tels que définis à l'une des revendications 1 à 3, caractérisé par le fait qu'il consiste :
   - à effectuer une hydrolyse enzymatique d'une matière première à base de pomme, à l'état hydraté, à l'aide d'une préparation enymatique contenant des activités pectinolytiques, hémicellulosiques et/ou cellulosiques ;
   - à séparer le produit de la réaction du milieu réactionnel ;
   - à en recueillir une fraction passant entre des tamis de 0,5 à 0,05 mm d'ouverture de maille ; puis,
   - à purifier éventuellement et à sécher cette fraction.

5. Procédé selon la revendication 4, caractérisé par le fait que l'an utilise, comme matière première a base de pomme, des résidus à teneur importante en macromolécules d'origine pariétale :
   - le marc, humide ou sec (que l'on réhydrate alors dans ce cas) ;
   - las rétentats d'ultrafiltration et de microfiltration, obtenus dans le traitement industriel des pommes ;
   - les gels de pectate de calcium (rétentats d'ultrafiltration et chapeaux bruns de défécation) : et
   - les résidus de pectinerie.

6. Procédé selon l'une des revendications 4 et 5, caractérisé par le fait que l'on utilise une préparation enzymatique qui contient des activités pectinestérase, polygalacturonase, cellulase et arabinogalactanase.

7. Procédé selon l'une des revendications 4 à 6, caractérisé par le fait qu'on ajuste le pH dans la zone de réaction enymatique à une valeur allant de 4 à 5.

8. Procédé selon l'une des revendications 4 à 7, caractérisé par le fait qu'on effectue l'hydrolyse enzymatique à une température inférieure à 50°C, notamment comprise entre 35 et 50°C.

9. Procédé selon l'une des revendications 4 à 8, caractérise par le fait que l'on recueille la fraction active par tamisages successifs de l'hydrolysat.

10. Procédé selon l'une des revendications 4 à 9, caractérisé par le fait que l'on recueille la fraction active par ultrafiltration.

11. Procédé selon l'une des revendications 4 à 10, caractérisé par le fait qu'on effectue la purification de la fraction active par dialyse par diafiltration, avec éventuellement addition d'agents chélateurs d'ions.

12. Utilisation des complexes macromoléculaires tels que définis à l'une des revendications 1 à 3, pour la fabrication d'agents hypocholestérolémiants.

13. Composition alimentaire ou diététique ou boisson renfermant une quantité des complexes macromoléculaires tels que définis à l'une des revendications 1 à 3, suffisante pour produire un effet hypocholestérolémiant.

14. Composition pharmaceutique hypocholestérolémiante renfermant une quantité efficace des complexes macromoléculaires tels que définis a l'une des revendications 1 à 3.

15. Médicament ayant un effet hypocholestérolémiant contenant des complexes macromoléculaires tels que définis à l'une des revendications 1 à 3.

EP 0 393 106 B1

**Claims**

1. Macromolecular complexes of vegetable origin, characterized in that they contain, on a dry basis,
   - from 5 to 25% by weight of cationic polysaccharides;
   - from 5 to 15% by weight of hemicelluloses;
   - from 0 to 50% by weight of cellulose;
   - from 25 to 55% by weight of polymerized quinonoid pigments.

2. Macromolecular complexes according to claim 1, characterized in that the cationic polysaccharides essentially consist of pectin derivatives.

3. Macromolecular complexes according to one of claims 1 and 2, characterized in that the preponderant monomers of the hemicelluloses are arabinose, galactose, glucose and mannose.

4. Process for preparing the macromolecular complexes of vegetable origin as defined in one of claims 1 to 3, characterized in that it consists in:
   - performing an enzymatic hydrolysis of a raw material based on apple, in the hydrated state, using an enzyme preparation containing pectinolytic, hemicellulose and/or cellulose activities;
   - separating the reaction product from the reaction medium;
   - collecting therefrom a fraction passing between sieves of mesh aperture 0.5 to 0.05 mm; and then
   - purifying, where appropriate, and drying this fraction.

5. Process according to claim 4, characterized in that, as a raw material based on apple, residues are used having a high content of macromolecules of parietal origin:
   - pomace, wet or dry (which is then rehydrated in this case);
   - ultrafiltration and microfiltration retentates, obtained in the industrial processing of apples;
   - calcium pectate gels (ultrafiltration retentates and brown heads of clarification); and
   - residues of pectin manufacture.

6. Process according to one of claims 4 and 5, characterized in that an enzyme preparation which contains pectinesterase, polygalacturonase, cellulase and arabinogalactanase activities is used.

7. Process according to one of claims 4 to 6, characterized in that the pH is adjusted within the zone of enzymatic reaction to a value ranging from 4 to 5.

8. Process according to one of claims 4 to 7, characterized in that the enzymatic hydrolysis is performed at a temperature below 50°C, in particular between 35 and 50°C.

9. Process according to one of claims 4 to 8, characterized in that the active fraction is collected by successive sievings of the hydrolysate.

10. Process according to one of claims 4 to 9, characterized in that the active fraction is collected by ultrafiltration.

11. Process according to one of claims 4 to 10, characterized in that the purification of the active fraction is performed by dialysis by diafiltration, with the optional addition of ion chelating agents.

12. Use of the macromolecular complexes as defined in one of claims 1 to 3, for the making of a hypocholesterolemic agent.

13. Foodstuff or dietary composition or drink containing an amount of the macromolecular complexes, as defined in one of claims 1 to 3, sufficient for producing a hypocholesterolemic effect.

14. Hypocholesterolemic pharmaceutic composition containing an effective amount of the macromolecular complexes as defined in one of claims 1 to 3.

15. Hypocholesterolemic medecine containing macromolecular complexes as defined in one of the claims 1 to 3.

9

**Patentansprüche**

1. Makromolekulare Komplexe pflanzlichen Ursprungs, dadurch gekennzeichnet, daß sie, bezogen auf das Trockengewicht, umfassen:

    5 bis 25 Gew.-% kationische Polyoside,

    5 bis 15 Gew.-% Hemicellulosen,

    0 bis 50 Gew.-% Cellulose,

    25 bis 55 Gew.-% polymerisierte Chinon-Pigmente.

2. Makromolekulare Komplexe nach Anspruch 1, dadurch gekennzeichnet, daß die kationischen Polyoside im wesentlichen aus Pectin-Derivaten bestehen.

3. Makromolekulare Komplexe nach einem der Ansprüche 1 und 2, dadurch gekennzeichnet, daß die überwiegenden Monomere der Hemicellulosen Arabinose, Galactose, Glucose und Mannose sind.

4. Verfahren zur Herstellung makromolekularer Komplexe pflanzlichen Ursprungs nach einem der Ansprüche 1 bis 3, dadurch gekennzeichnet, daß es besteht in
   - der Durchführung einer enzymatischen Hydrolyse eines Rohstoffs auf Apfelbasis in wasserhaltigem Zustand mit Hilfe eines Enzympräparats mit pectinolytischer Wirkung, Hemicellulose- und/oder Cellulose-Aktivität,
   - der Abtrennung des Reaktionsprodukts aus dem Reaktionsgemisch,
   - der Gewinnung einer Fraktion daraus, die ein Sieb mit 0,5 bis 0,05 mm Maschenweite passiert, und
   - gegebenenfalls der Reinigung und Trocknung dieser Fraktion.

5. Verfahren nach Anspruch 4, dadurch gekennzeichnet, daß man als Rohstoff auf Apfelbasis Rückstände mit bedeutenden Gehalten an Makromolekülen parietalen (wandständigen) Ursprungs verwendet:
   - Trester, feucht oder trocken (die dann in diesem Falle rehydratisiert werden),
   - Filtriergut aus der Ultrafiltration und der Mikrofiltration, erhalten bei der industriellen Äpfelverwertung,
   - Calciumpectat-Gele (Filtriergut aus der Ultrafiltration und braune Klärschlämme), und
   - Rückstände der Pectin-Verwertung.

6. Verfahren nach einem der Ansprüche 4 und 5, dadurch gekennzeichnet, daß man ein Enzympräparat verwendet, das Pectinesterase-, Polygalacturonase-, Cellulase- und Arabinogalactanase-Wirkung besitzt.

7. Verfahren nach einem der Ansprüche 4 bis 6, dadurch gekennzeichnet, daß der pH-Wert im Gebiet der Enzymreaktion auf einen Wert von 4 bis 5 eingestellt wird.

8. Verfahren nach einem der Ansprüche 4 bis 7, dadurch gekennzeichnet, daß die enzymatische Hydrolyse bei einer Temperatur unterhalb 50°C durchgeführt wird, insbesondere von einschließlich 35 bis 50°C.

9. Verfahren nach einem der Ansprüche 4 bis 8, dadurch gekennzeichnet, daß die wirksame Fraktion durch sukzessives Durchsieben des Hydrolysats gewonnen wird.

10. Verfahren nach einem der Ansprüche 4 bis 9, dadurch gekennzeichnet, daß die wirksame Fraktion durch Ultrafiltration gewonnen wird.

11. Verfahren nach einem der Ansprüche 4 bis 10, dadurch gekennzeichnet, daß die Reinigung der wirksamen Fraktion mittels Diafiltrationsdialyse, gegebenenfalls unter Zusatz von Chelatbildnern durchgeführt wird.

12. Verwendung der makromolekularen Komplexe nach einem der Ansprüche 1 bis 3 als Hypocholesterinämie auslösendes Mittel.

13. Lebensmittel- oder Diät- oder Trinkzubereitung, umfassend eine Menge makromolekularer Komplexe nach einem der Ansprüche 1 bis 3, die ausreicht, um einen hypocholesterinämischen Effekt hervorzurufen.

14. Pharmazeutische Zubereitung mit hypocholesterinämischer Wirkung, die eine wirksame Menge makromolekularer Komplexe nach einem der Ansprüche 1 bis 3 umfaßt.

15. Medikament mit hypochoesterinämischer Wirkung, das makromolekulare Komplexe nach einem der Ansprüche 1 bis 3 enthält.